Europäisches Patentamt

European Patent Office

Office européen des brevets

(19)

(11) Veröffentlichungsnummer : **0 066 169**
**B1**

(12)

# EUROPÄISCHE PATENTSCHRIFT

(45) Veröffentlichungstag der Patentschrift :
30.01.85

(51) Int. Cl.⁴ : **C 07 C 57/02, C 07 C 51/09**

(21) Anmeldenummer : **82104303.1**

(22) Anmeldetag : **17.05.82**

(54) Verfahren zur Herstellung ungesättigter aliphatischer Carbonsäuren.

(30) Priorität : **21.05.81 DE 3120244**

(43) Veröffentlichungstag der Anmeldung :
**08.12.82 Patentblatt 82/49**

(45) Bekanntmachung des Hinweises auf die Patenterteilung : **30.01.85 Patentblatt 85/05**

(84) Benannte Vertragsstaaten :
**CH DE FR GB LI**

(56) Entgegenhaltungen :
**DE-A- 2 526 716**
**DE-C- 1 059 899**

(73) Patentinhaber : **HOECHST AKTIENGESELLSCHAFT**
**Postfach 80 03 20**
**D-6230 Frankfurt am Main 80 (DE)**

(72) Erfinder : **Neu, Hermann, Dr.**
**Wilhelmstrasse 14**
**D-6078 Neu-Isenburg (DE)**
Erfinder : **Roscher, Günter, Dr.**
**Altkönigstrasse 7**
**D-6233 Kelkheim (taunus) (DE)**

**Beschreibung**

Die Erfindung betrifft ein Verfahren zur Herstellung ungesättigter aliphatischer Carbonsäuren durch thermische Spaltung des bei der katalytischen Umsetzung von Aldehyden oder Ketonen mit Keten gebildeten polymeren Zwischenprodukts (im folgenden « Polyester » genannt). Ein solches Verfahren ist. z. B. in US-PS 3 021 365 beschrieben.

Als Katalysatoren für die Umsetzung zum Polyester eignen sich solche vom Friedel-Crafts-Typ, wie z. B. Borfluorid, Aluminiumchlorid und Zinkchlorid, aber auch zweiwertige Metalle wie Zink, Cadmium, Quecksilber, Nickel, Cobalt, Eisen in Form ihrer Carboxylate. Die Reaktionstemperatur liegt je nach eingesetztem Aldehyd bzw. Keton (im folgenden « Carbonylverbindung » genannt) bei 20-60 °C. Es ist zweckmäßig, die Reaktion in einem inerten Lösungsmittel, wie z. B. Toluol, ablaufen zu lassen oder die Carbonylverbindung nur zum Teil umzusetzen, so daß der Überschuß als Lösungsmittel fungiert. Vor der Weiterverarbeitung des Polyesters müssen das Lösungsmittel und die nicht umgesetzte Carbonylverbindung abdestilliert werden.

Die thermische Spaltung des Polyesters wird vorzugsweise bei Temperaturen von 150-250 °C, insbesondere bei 170-200 °C, in Gegenwart katalytischer Mengen von Alkalihydroxiden oder basisch reagierenden Alkalisalzen oder sekundären oder tertiären Aminen durchgeführt. Im allgemeinen wird die gebildete Carbonsäure gleichzeitig abdestilliert. Falls die gewünschten ungesättigten Carbonsäuren im Normalzustand fest sind, ist es zweckmäßig, die thermische Spaltung des Polyesters und die anschließende gleichzeitige Destillation der Säure in einem hochsiedenden inerten Lösungsmittel vorzunehmen.

Auf diese Weise können z. B. ungesättigte aliphatische Carbonsäuren mit 4 bis 16 C-Atomen (einfach oder mehrfach ungesättigt) aus den entsprechenden Carbonylverbindungen hergestellt werden. Insbesondere können die folgenden ungesättigten aliphatischen Carbonsäuren aus den jeweils angegebenen Carbonylverbindungen hergestellt werden, indem man letztere mit Keten zum entsprechenden Polyester umsetzt und diesen thermisch spaltet : Hexensäure aus Butyraldehyd, Ethylhexensäure aus Ethylbutyraldehyd, Ethyloctensäure aus Ethylhexanal, i-Undecensäure aus i-Nonylaldehyd, Heptensäure aus Valeraldehyd, Hexadecensäure aus Myristinaldehyd, Pentadiensäure aus Acrolein, Hexadiensäure aus Crotonaldehyd, Dimethylacrylsäure aus Aceton, Methylpentensäure aus Methylethylketon. Besonders geeignet ist das Verfahren für die Herstellung von Hexensäure, Ethylhexensäure, i-Undecensäure, Hexadiensäure.

Bei der thermischen Spaltung des Polyesters entstehen neben den ungesättigten Säuren auch höhersiedende flüssige Produkte, die keine Säureeigenschaften besitzen. Diese Nebenprodukte können die Ausbeute an ungesättigter Carbonsäure erheblich vermindern und zu einer Verfärbung führen.

Es wurde überraschenderweise gefunden, daß durch Erhitzen des Polyesters mit Wasser vor der thermischen Spaltung die Bildung dieser Hochsieder zurückgedrängt, die Ausbeute an ungesättigter Säure erhöht und deren Qualität wesentlich verbessert wird.

Das erfindungsgemäße Verfahren zur Herstellung von ungesättigten aliphatischen Carbonsäuren durch thermische Spaltung des bei der Kondensation von Carbonylverbindungen mit Keten, ggf. in Anwesenheit eines Lösungsmittels, gebildeten Polyesters, ist dadurch gekennzeichnet, daß das polyesterhaltige Reaktionsgemisch oder der hieraus isolierte Polyester nach Zusatz von Wasser auf eine Temperatur erhitzt wird, die gleich dem Siedepunkt ist oder weniger als 40 °C unterhalb des Siedepunkts liegt, bevor man den Polyester nach Isolation, spaltet. Vorzugsweise arbeitet man am Siedepunkt oder weniger als 20 °C unterhalb des Siedepunkts, insbesondere aber am Siedepunkt selbst.

Die Temperatur liegt also bei 60-100 °C, vorzugsweise bei 80-100 °C, insbesondere bei etwa 100 °C, wenn bei Normaldruck gearbeitet wird. Die Erhitzung mit Wasser kann jedoch auch unter Druck oder Vakuum ausgeführt werden, wobei sich der Siedepunkt und die Lage des 40 Grad breiten Temperaturintervalls entsprechend verschiebt. Jedoch ist das Arbeiten unter Normaldruck bevorzugt. Vorzugsweise arbeitet man außerdem am Rückfluß.

Die Menge des zugesetzten Wasser kann in weiten Grenzen variiert werden. Im allgemeinen genügt ein Wasserzusatz von 5-20 Gew.-%, bezogen auf den eingesetzten Polyester, doch sind auch größere Zusatzmengen möglich, ohne daß der beschriebene Effekt beeinflußt wird. Die Dauer der Wasserbehandlung ist von dem eingesetzten Polyester abhängig, sie liegt in der Regel zwischen 30 und 60 Minuten. Eine längere Erhitzungsdauer hat keinen negativen Einfluß auf die Ausbeute, sie bringt aber auch keine weiteren Vorteile. Das Erhitzen in Gegenwart von Wasser kann sowohl vor wie nach dem Abdestillieren der Leichtsieder, der nicht umgesetzten Carbonylverbindung und ggf. des Lösungsmittels erfolgen. Vorzugsweise erfolgt es vorher.

Eine besonders günstige Arbeitsweise ist es, nach der Umsetzung der Carbonylverbindung mit Keten das Wasser und den Katalysator für die Spaltung des Polyesters zu dem polyesterhaltigen Reaktionsgemisch zu geben und unter Normaldruck am Rückfluß zu kochen. Anschließend werden Leichtsieder, nicht umgesetzte Carbonylverbindung, Wasser und Lösungsmittel abdestilliert. Danach wird der Sumpf, der im wesentlichen aus Polyester besteht, auf die zur Spaltung erforderliche Temperatur von 150-200 °C gebracht. Unter Vakuum wird die ungesättigte Carbonsäure entsprechend ihrer Bildungsgeschwindigkeit abdestilliert. Auch ohne Verwendung einer Kolonne fällt bei einfachem Überdestillieren die ungesättigte Säure in so hoher Reinheit an, daß sich eine weitere Reinigungsoperation, etwa durch wiederholtes Destillieren, erübrigt. Ohne die erfindungsgemäße Erhitzung mit Wasser kann eine solche Qualität der Säure nicht so leicht erreicht werden, denn selbst eine Destillation über eine Kolonne bei hohem Rücklaufverhältnis liefert immer noch schwach gelb gefärbte Produkte, abgesehen davon, daß diese Maßnahme die Ausbeute erheblich verschlechtert.

Die nachfolgenden Beispiele erläutern die Erfindung.

Vergleichsbeispiel 1

a) In ein Gemisch aus 250 g Butyraldehyd, 250 g Toluol und 7,5 g Zinkisobuyrat wird bei 30-40 °C Keten eingeleitet, bis die Gewichtszunahme einem Umsatz von 80-90 % des eingesetzten Aldehyds entspricht. Nach der Reaktion wird das polyesterhaltige Gemisch mit 5 g Dioctadecyl-amin versetzt.

b) Nun werden die Leichtsieder, der nicht umgesetzte Butyraldehyd und das Toluol abdestilliert. Anschließend wird unter einem Vakuum von 25 mbar und Steigerung der Sumpftemperatur auf 170-200 °C der Polyester gespalten und die Hexensäure laufend abdestilliert. Es werden 205 g Hexensäure ($Kp_{25}$ 117 °C) erhalten, das entspricht einer Ausbeute von 61,0 %, bezogen auf umgesetzten Butyraldehyd. Die Lichtabsorption bei 415 nm in 1 cm Schnichtdicke im Vergleich zu Wasser beträgt 89 %.

Beispiel 1

a) Man verfährt wie in Teil a) des Vergleichsbeispiels 1.

b) Das Gemisch wird mit 40 g Wasser versetzt und 30 Minuten lang am Rückfluß zum Sieden erhitzt. Anschließend werden die Leichtsieder, der nicht umgesetzte Butyraldehyd, das Wasser und das Toluol abdestilliert. Dann wird bei einem Vakuum von 25 mbar und Steigerung der Sumpftemperatur auf 170-200 °C der Polyester gespalten und die Hexensäure laufend abdestilliert. Es werden 291 g Hexensäure ($Kp_{25}$ 117 °C) erhalten, das entspricht einer Ausbeute von 86,4 %, bezogen auf umgesetzten Butyraldehyd. Die Lichtabsorption bei 415 nm in 1 cm Schichtdicke im Vergleich zu Wasser beträgt 34 %.

Vergleichsbeispiel 2

a) Man verfährt wie in Teil a) des Vergleichsbeispiels 1, nur daß statt Butyraldehyd dieselbe Menge Propionaldehyd eingesetzt wird.

b) Man verfährt analog zu Teil b) des Vergleichsbeispiels 1. Es werden 209 g Pentensäure ($Kp_{27}$ 107 °C) erhalten, das enspricht einer Ausbeute von 60,6 %, bezogen auf umgesetzten Propionaldehyd.

Beispiel 2

a) Man verfährt wie in Teil a) des Vergleichsbeispiels 2.

b) Man verfährt analog zu Teil b) des Beispiels 1, nur daß man vor der Spaltung das mit Wasser versetzte Gemisch auf 120 °C (bei einem entsprechenden Überdruck) erhitzt. Es werden 272 g Pentensäure ($Kp_{27}$ 107 °C) erhalten, das entspricht einer Ausbeute von 78,8 %, bezogen auf umgesetzten Priopionaldehyd.

Vergleichsbeispiel 3

a) Man verfährt wie in Teil a) des Vergleichsbeispiels 1, nur daß statt Butyraldehyd dieselbe Menge 2-Ethylbutanal eingesetzt wird.

b) Man verfährt analog zu Teil b) des Vergleichsbeispiels 1. Es werden 214 g 4-Ethylhexensäure ($Kp_{25}$ 139-141 °C) erhalten, das entspricht einer Ausbeute von 69,3 %, bezogen auf umgesetztes Ethylbutanal.

Beispiel 3

a) Man verfährt wie in Teil a) des Vergleichsbeispiels 3.

b) Man verfährt analog zu Teil b) des Beispiels 1, nur daß man vor der Spaltung 50 Minuten bei 90 °C und entsprechendem Unterdruck mit dem zugesetzten Wasser am Rückfluß kocht. Es werden 260 g 4-Ethylhexensäure ($Kp_{25}$ 139-141 °C) erhalten, das entspricht einer Ausbeute von 84,1 %, bezogen auf umgesetztes 2-Ethylbutanal.

Vergleichsbeispiel 4

a) Man verfährt wie in Teil a) des Vergleichsbeispiels 1, nur daß statt Butyraldehyd dieselbe Menge Valeraldehyd eingesetzt wird.

b) Man verfährt analog zu Teil b) des Vergleichsbeispiels 1. Es werden 198 g Heptensäure ($Kp_{25}$ 139-141 °C) erhalten, das entspricht einer Ausbeute von 62,6 %, bezogen auf umgesetzten Valeraldehyd.

Beispiel 4

a) Man verfährt wie in Teil a) des Vergleichsbeispiels 4.

b) Man verfährt analog zu Teil b) des Beispiels 1, nur daß man (vor der Spaltung) mit 50 g Wasser auf 90 °C für 60 Minuten erhitzt. Es werden 268 g Heptensäure ($Kp_{25}$ 139-141 °C) erhalten, das entspricht einer Ausbeute von 84,8 %, bezogen auf umgesetzten Valeraldehyd.

Vergleichsbeispiel 5

a) Man verfährt wie in Teil a) des Vergleichsbeispiels 1, nur daß statt Butyraldehyd dieselbe Menge i-Nonylaldehyd eingesetzt werden.

b) Man verfährt analog zu Teil b) des Vergleichsbeispiels 1. Es werden 174 g i-Undecensäu-

re (Kp$_{25}$ 163-166 °C) erhalten, das entspricht einer Ausbeute von 63,2 %, bezogen auf umgesetzten i-Nonylaldehyd.

Beispiel 5

a) Man verfährt wie in Teil a) des Vergleichsbeispiels 5.

b) Man destilliert den nicht umgesetzten Aldehyd und das Toluol ab, versetzt den Rückstand mit 40 g Wasser und erhitzt 30 Minuten lang zum Sieden. Anschließend wird das Wasser abdestilliert und der Rückstand thermisch gespalten. Es werden 219 g i-Undecensäure erhalten, das entspricht einer Ausbeute von 79,5 %, bezogen auf umgesetzten i-Nonylaldehyd.

Vergleichsbeispiel 6

a) In ein Gemisch aus 250 g Methylethylketon, 250 g Toluol und 2,5 ml 30 %iger Bortrifluoridlösung in Diethylether wird bei 20-25 °C Keten eingeleitet, bis die Gewichtszunahme einem Umsatz von 80-90 % des eingesetzten Ketons entspricht. Nach der Reaktion wird das polyesterhaltige Gemisch mit 5 g Dimethylstearylamin versetzt.

b) Nun werden die Leichtsieder, das nicht umgesetzte Methylethylketon und das Toluol abdestilliert. Anschließend wird bei einem Vakuum von 25 mbar und Steigerung der Sumpftemperatur auf 170-190 °C der Polyester gespalten und die 3-Methylpentensäure (Kp$_{25}$ 117-121 °C) erhalten, das entspricht einer Ausbeute von 60,1 %, bezogen auf umgesetztes Methylethylketon.

Beispiel 6

a) Man verfährt wie in Teil a) des Vergleichsbeispiels 6.

b) Das Gemisch wird mit 50 g Wasser 60 Minuten lang am Rückfluß gekocht. Anschließend werden die Leichtsieder, das nicht umgesetzte Methylethylketon, das Wasser und das Toluol abdestilliert. Der Rückstand wird bei einem Vakuum von 25 mbar und einer Sumpftemperatur von 170-190 °C gespalten, unter gleichzeitiger Destillation der 3-Methylpentensäure (Kp$_{25}$ 117-121 °C). Es werden 268 g 3-Methylpentensäure erhalten, das entspricht einer Ausbeute von 79,8 %, bezogen auf umgesetztes Methylethylketon.

**Ansprüche**

1. Verfahren zur Herstellung von ungesättigten aliphatischen Carbonsäuren durch thermische Spaltung des bei der Kondensation von Carbonylverbindungen mit Keten, gebildeten Polyesters, dadurch gekennzeichnet, daß das polyesterhaltige Reaktionsgemisch oder der hieraus isolierte Polyester nach Zusatz von Wasser auf eine Temperatur erhitzt wird, die gleich dem Siedepunkt ist oder weniger als 40 °C unterhalb des Siedepunkts liegt, bevor man den Polyester nach Isolation spaltet.

2. Verfahren nach Anspruch 1, dadurch gekennzeichnet, daß nach Zusatz von Wasser auf eine Temperatur erhitzt wird, die gleich dem Siedepunkt ist oder weniger als 20 °C unterhalb des Siedepunkts liegt.

3. Verfahren nach Anspruch 1 oder 2, dadurch gekennzeichnet, daß das polyesterhaltige Reaktionsgemisch nach Zusatz von Wasser erhitzt wird und dann die Leichtsieder, die nicht umgesetzte Carbonylverbindung und das Wasser abgetrennt werden, bevor man den auf diese Weise isolierten Polyester spaltet.

4. Verfahren zur Herstellung von ungesättigten aliphatischen Carbonsäuren durch thermische Spaltung des bei der Kondensation von Carbonylverbindungen mit Keten in Anwesenheit eines Lösungsmittels, gebildeten Polyesters, dadurch gekennzeichnet, daß das polyesterhaltige Reaktionsgemisch oder der hieraus isolierte Polyester nach Zusatz von Wasser auf eine Temperatur erhitzt wird, die gleich dem Siedepunkt ist oder weniger als 40 °C unterhalb des Siedepunkts liegt, bevor man den Polyester nach Isolation spaltet.

5. Verfahren nach Anspruch 4, dadurch gekennzeichnet, daß nach Zusatz von Wasser auf eine Temperatur erhitzt wird, die gleich dem Siedepunkt ist oder weniger als 20 °C unterhalb des Siedepunkts liegt.

6. Verfahren nach Anspruch 4 oder 5, dadurch gekennzeichnet, daß das polyesterhaltige Reaktionsgemisch nach Zusatz von Wasser erhitzt wird und dann die Leichtsieder, die nicht umgesetzte Carbonylverbindung, das Wasser und das Lösungsmittel abgetrennt werden, bevor man den auf diese Weise isolierten Polyester spaltet.

**Claims**

1. A process for the preparation of unsaturated aliphatic carboxylic acids by thermal cracking of the polyester formed in the condensation reaction of a carbonyl compound with ketene, characterized by heating the reaction mixture containing polyester, or the polyester isolated therefrom, after adding water, to a temperature which is the same as the boiling point or less than 40 °C below the boiling point, before the polyester, after isolation, is subjected to cracking.

2. The process as claimed in claim 1, wherein, after adding water, the mixture is heated to a temperature which is the same as the boiling point or is less than 20 °C below the boiling point.

3. The process as claimed in claim 1 or 2, wherein, after adding water, the reaction mixture containing polyester is heated, and the lowboilers, the unreacted carbonyl compound and the water are then removed before the polyester which has been isolated in this manner is subjected to cracking.

4. A process for the preparation of an unsaturated aliphatic carboxylic acid by thermal cracking of the polyester formed in the condensation reaction of a carbonyl compound with ketene in the presence of a solvent, which comprises heating the reaction mixture containing polyester, or the polyester isolated therefrom, after adding water, to a temperature which is the same as the boiling point or is less than 40 °C below the boiling point, before the polyester, after isolation, is subjected to cracking.

5. The process as claimed in claim 4, wherein, after adding water, the mixture is heated to a temperature which is the same as the boiling point or is less than 20 °C below the boiling point.

6. The process as claimed in claim 4 or 5, wherein, after adding water, the reaction mixture containing polyester is heated, and the low-boilers, the unreacted carbonyl compound, the water and the solvent, are then removed before the polyester which has been isolated in this manner is subjected to cracking.

## Revendications

1. Procédé de préparation d'acides carboxyliques aliphatiques insaturés par coupure thermique du polyester formé lors de la condensation de composés carbonyliques avec le cétène, procédé caractérisé en ce que le mélange réactionnel contenant le polyester, ou le polyester isolé à partir de ce mélange, est chauffé, après avoir été additionné d'eau, à une température égale au point d'ébullition ou inférieure d'au plus 40 °C au point d'ébullition, avant que le polyester, préalablement isolé, soit soumis à la réaction de coupure.

2. Procédé selon la revendication 1, caractérisé en ce que, après avoir ajouté de l'eau, on chauffe à une température qui est égale au point d'ébullition ou qui est inférieure d'au plus 20 °C au point d'ébullition.

3. Procédé selon l'une des revendications 1 et 2, caractérisé en ce qu'on chauffe le mélange réactionnel contenant le polyester, après y avoir ajouté de l'eau, puis on chasse les constituants volatils, le composé carbonylique qui n'a pas réagi et l'eau, après quoi on soumet à la réaction de coupure le polyester qui a ainsi été isolé.

4. Procédé de préparation d'acides carboxyliques aliphatiques insaturés par coupure thermique du polyester formé lors de la condensation de composés carbonyliques avec le cétène en présence d'un solvant, procédé caractérisé en ce que le mélange réactionnel contenant le polyester, ou le polyester isolé à partir de ce mélange, est chauffé, après avoir été additionné d'eau, à une température égale au point d'ébullition ou inférieure d'au plus 40 °C au point d'ébullition, avant que le polyester, préalablement isolé, soit soumis à la réaction de coupure.

5. Procédé selon la revendication 4, caractérisé en ce que, après avoir ajouté de l'eau, on chauffe à une température qui est égale au point d'ébullition ou qui est inférieure d'au plus 20 °C au point d'ébullition.

6. Procédé selon l'une des revendications 4 et 5, caractérisé en ce qu'on chauffe le mélange réactionnel contenant le polyester, après y avoir ajouté de l'eau, puis on chasse les constituants volatils, le composé carbonylique qui n'a pas réagi, l'eau et le solvant, après quoi on soumet à la réaction de coupure le polyester qui a ainsi été isolé.